(19) Europäisches Patentamt
European Patent Office
Office européen des brevets

(11) **EP 2 323 973 B1**

(12) **EUROPÄISCHE PATENTSCHRIFT**

(45) Veröffentlichungstag und Bekanntmachung des Hinweises auf die Patenterteilung:
**21.03.2012 Patentblatt 2012/12**

(21) Anmeldenummer: **09781542.7**

(22) Anmeldetag: **06.08.2009**

(51) Int Cl.:
***C07C 263/10*** *(2006.01)*

(86) Internationale Anmeldenummer:
**PCT/EP2009/060184**

(87) Internationale Veröffentlichungsnummer:
**WO 2010/015667 (11.02.2010 Gazette 2010/06)**

(54) **VERFAHREN ZUR HERSTELLUNG VON AROMATISCHEN ISOCYANATEN**

METHOD FOR PRODUCING AROMATIC ISOCYANATES

PROCEDE DE FABRICATION D'ISOCYANATES AROMATIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **07.08.2008 EP 08161976**

(43) Veröffentlichungstag der Anmeldung:
**25.05.2011 Patentblatt 2011/21**

(73) Patentinhaber: **BASF SE**
**67056 Ludwigshafen (DE)**

(72) Erfinder:
- **PENZEL, Ulrich**
  **01945 Tettau (DE)**
- **MATTKE, Torsten**
  **67251 Freinsheim (DE)**
- **WÖLFERT, Andreas**
  **74906 Bad Rappenau (DE)**
- **JACOBS, Johannes**
  **4641 PE Ossendrecht (NL)**
- **CHENG, Tsung-Chieh**
  **64646 Heppenheim (DE)**
- **HEINEN, Kerstin**
  **64653 Lorsch (DE)**
- **STROEFER, Eckhard**
  **68163 Mannheim (DE)**
- **MAIXNER, Stefan**
  **68723 Schwetzingen (DE)**
- **MACKENROTH, Wolfgang**
  **67098 Bad Dürkheim (DE)**
- **TRAGUT, Christian**
  **1933 Sterrebeck (BE)**
- **THIELE, Kai**
  **2040 Antwerpen 4 (BE)**
- **WON, Myung Un**
  **Soho dong yeosu city (KR)**

(56) Entgegenhaltungen:
DD-A- 300 168      US-A1- 2005 113 601
US-A1- 2007 043 233      US-A1- 2008 027 242

**Beschreibung**

Die Erfindung betrifft ein Verfahren zur Herstellung von Isocyanaten durch Umsetzung der korrespondierenden Amine mit Phosgen in der Flüssigphase, gegebenenfalls in Gegenwart mindestens eines Inertmediums, bei dem zunächst das Amin und das Phosgen in einer Mischkammer zu einem Reaktionsgemisch gemischt werden und das Reaktionsgemisch einem Reaktor zugeführt wird. Das Amin wird durch eine koaxial zur Mischkammer angeordnete Öffnung zugegeben und das Phosgen durch Zufuhröffnungen in mindestens zwei senkrecht zur Achse der Mischkammer angeordneten Ebenen. Mindestens eine Ebene ist in Hauptströmungsrichtung des Reaktionsgemischs stromauf und eine Ebene stromab zur Öffnung der Zugabe des Amins angeordnet.

Die Herstellung von Isocyanaten durch Phosgenierung der korrespondierenden Amine kann prinzipiell durch eine FLüssigphasen- oder eine Gasphasenphosgenierung erfolgen. Die Flüssigphasenphosgenierung zeichnet sich dadurch aus, dass die Reaktion bei niedrigeren Temperaturen als die Gasphasenphosgenierung durchgeführt werden kann und eine Verdampfung der Edukte nicht erforderlich ist.

Bei der Flüssigphasenphosgenierung wird ein aminhaltiger Eduktstrom in der Flüssigphase zugeführt. Dieser wird mit einem phosgenhaltigen Eduktstrom vermischt. Hierbei kann das Phosgen in einem inerten Lösungsmittel gelöst sein. Anschließend wird der phosgenhaltige Eduktstrom in eine Mischkammer eingespritzt, in der dieser mit dem aminhaltigen Eduktstrom vermischt wird. Das Amin und das Phosgen setzen sich unter Freisetzung von HCl zu den entsprechenden Isocyanaten um.

Eine schnelle Vermischung des Amins mit dem Phosgen ist notwendig, da das entstehende Isocyanat bei einer zu niedrigen Phosgenkonzentration mit dem überschüssigen Amin zu Harnstoff bzw. anderen störenden, hochviskosen und festen Nebenprodukten reagiert. Aus diesem Grund ist eine schnelle Vermischung und eine kurze Verweilzeit in der Reaktionskammer erforderlich.

Eine Vorrichtung, bei der das Amin und das Phosgen zunächst in einer Mischkammer zu einem Reaktionsgemisch gemischt werden und das Reaktionsgemisch anschließend einem Reaktor zugeführt wird, wobei das Amin durch eine koaxial zur Mischkammer angeordnete Öffnung zugegeben wird und das Phosgen durch Zufuhröffnungen in mindestens zwei senkrecht zur Achse der Mischkammer angeordneten Ebenen zugegeben wird, ist zum Beispiel aus DD-A 300 168 bekannt.

Aufgabe der vorliegenden Erfindung ist es, ein Verfahren zur Herstellung von Isocyanaten durch Umsetzung der korrespondierenden Amine mit Phosgen in der Flüssigphase bereitzustellen, bei dem eine im Vergleich zu den aus dem Stand der Technik bekannten Verfahren geringere Nebenkomponentenbildung erreicht werden kann.

Gelöst wird die Aufgabe durch ein Verfahren zur Herstellung von Isocyanaten durch Umsetzung der korrespondierenden Amine mit Phosgen in der Flüssigphase, gegebenenfalls in Gegenwart mindestens eines Inertmediums, bei dem zunächst das Amin und das Phosgen in einer Mischkammer zu einem Reaktionsgemisch gemischt werden und das Reaktionsgemisch einem Reaktor zugeführt wird. Das Amin wird durch eine koaxial zur Mischkammer angeordnete Öffnung zugegeben und das Phosgen wird durch Zufuhröffnungen in mindestens zwei senkrecht zur Achse der Mischkammer angeordneten Ebenen zugegeben. Dabei ist mindestens eine Ebene in Hauptströmungsrichtung des Reaktionsgemischs stromauf und eine Ebene stromab zur Öffnung der Zugabe des Amins angeordnet. Die mittlere Verweilzeit des Reaktionsgemischs in der Mischkammer beträgt erfindungsgemäß maximal 18,5 ms.

Durch die kurze Verweilzeit des Reaktionsgemischs in der Mischkammer von maximal 18,5 ms kann eine im Vergleich zu den aus dem Stand der Technik bekannten Verfahren reduzierte Nebenkomponentenbildung erzielt werden.

Die mittlere Verweilzeit in der Mischkammer ergibt sich dabei aus

$$t_s = \frac{V}{V*}.$$

Darin bedeuten $t_s$ die Verweilzeit, V das Volumen der Mischkammer und V* den Gesamtvolumenstrom der Eduktströme. Das Volumen der Mischkammer ist dabei das Volumen bis zum Ende der Verengung, das heißt, bis zum Eintritt in die sich an die Mischkammer anschließende Zone mit konstantem Querschnitt. Das Volumen der zentralen Düse, die in die Mischkammer ragt ist nicht Teil des Volumens der Mischkammer.

Das zur Herstellung von Isocyanaten eingesetzte Amin ist zum Beispiel ein Monoamin, ein Diamin, ein Triamin oder höherwertiges Amin. Bevorzugt werden jedoch Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Die Amine und Isocyanate können aliphatisch, cycloaliphatisch oder aromatisch sein. Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate stehen im Rahmen dieser Anmeldung kurz für cycloaliphatische und/oder aliphatische Isocyanate.

Beispiele für aromatische Diisocyanate sind monomeres 2,4'- oder 4,4'-Methylen-di(phenylisocyanat) (MDI) sowie dessen höhere Oligomere (PMDI) bzw. Mischungen daraus, 2,4- und/oder 2,6-Toluylendiisocyanat (TID) und 1,5- oder 1,8-Naphthyldiisocyanat (NDI).

Bevorzugte (cyclo)aliphatische Diisocyanate sind solche mit 4 bis 20 C-Atomen.

Beispiele für übliche aliphatische Diisocyanate sind 1,4-Tetramethylendiisocyanat, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanats, Tetramethylxylylendiisocyanat (TMXDI), Trimethylhexandiisocyanat oder Tetramethylhexandiisocyanat, sowie 3 (bzw. 4)-, 8 (bzw. 9)-Bis(isocyanatomethyl)-tricyclo[5.2.1.0$^{2,6}$]decan-Isomerengemische, sowie cycloaliphatische Diisocyanate, wie 1,4-, 1,3- oder 1,2-Diisocyanatocyclohexan, 4,4'- oder 2,4'-Di(isocyanatocyclo-hexyl)methan, 1-Isocyanato-3,3,5-trimethyl-5-(isocyanatomethyl)cyclohexan (Isophorondiisocyanat), 1,3- oder 1,4-bis(Isocyanatomethyl)cyclohexan, 2,4-, oder 2,6-Diiso-cyanato-1-methylcyclohexan.

Besonders bevorzugt sind MDI/PMDI-Isomeren- und -Oligomerengemische sowie TDI-Isomerengemische.

Zur Herstellung von Monoisocyanaten können ebenfalls aliphatische, cycloaliphatische oder aromatische Amine eingesetzt werden. Als aromatisches Amin ist insbesondere Anilin bevorzugt.

Das Phosgen kann vor Zugabe in die Mischkammer in einem inerten Lösungsmittel gelöst sein. Als inerte Lösungsmittel, in denen das Phosgen gelöst wird, eignen sich zum Beispiel chlorierte aromatische Kohlenwasserstoffe, beispielsweise Monochlorbenzol oder Dichlorbenzol, oder auch Toluol. Das Verhältnis von Phosgen zu inertem Lösungsmittel liegt bevorzugt im Bereich von 1:0 bis 1:2, insbesondere im Bereich von 1:0 bis 1:1.

In einer bevorzugten Ausführungsform wird das Phosgen über jeweils mindestens zwei Zufuhröffnungen in den mindestens zwei senkrecht zur Achse der Mischkammer angeordneten Ebenen zugegeben. Die Zufuhröffnungen, durch die das Phosgen zugegeben wird, sind dabei vorzugsweise so angeordnet, dass sich die Hauptrichtungen der Zufuhröffnungen in der Achse der Mischkammer treffen. Durch die Anordnung der Zufuhröffnungen derart, dass sich die Hauptrichtungen in der Achse der Mischkammer treffen, treffen die über die Zufuhröffnungen zugegebenen Phosgenstrahlen direkt auf das Amin, das durch die koaxial zur Mischkammer angeordnete Öffnung zugegeben wird. Hierdurch wird eine schnelle Vermischung von Phosgen und Amin realisiert. Insbesondere treffen auch Phosgenstrahlen, die die Zufuhröffnungen verlassen, an der Achse der Mischkammer zusammen. Hierdurch ergibt sich in Strömungsrichtung des Amins eine gleichmäßige Phosgenverteilung.

Weiterhin ist es bevorzugt, wenn die Zufuhröffnungen der ersten Ebene zu den Zufuhröffnungen der zweiten Ebene um die Achse der Mischkammer gedreht angeordnet sind. Besonders bevorzugt ist es, wenn die Zufuhröffnungen bei jeweils zwei Zufuhröffnungen pro Ebene um 90 Grad zueinander gedreht angeordnet sind.

Die Mischkammer, in der das Amin mit dem Phosgen gemischt wird, weist vorzugsweise ein Verhältnis von Länge zu Durchmesser (UD-Verhältnis) auf, das im Bereich von 1 bis 2 und insbesondere im Bereich von 1 bis 1,5 liegt. Die koaxial zur Achse der Mischkammer angeordnete Öffnung, über die das Amin zugegeben wird, ragt vorzugsweise in die Mischkammer hinein. Hierzu ist die Öffnung, über die das Amin zugegeben wird, zum Beispiel als Düse ausgebildet. Die Öffnung, über die das Amin zugegeben wird, ist dann die Austrittsöffnung der Düse. Das Verhältnis des Durchmessers der Öffnung, über die das Amin zugegeben wird bezogen auf den Durchmesser der Mischkammer liegt vorzugsweise im Bereich von 0,05 bis 0,5, mehr bevorzugt im Bereich von 0,1 bis 0,4 und insbesondere im Bereich von 0,15 bis 0,35.

Wenn das Phosgen durch Zufuhröffnungen in zwei senkrecht zur Achse der Mischkammer angeordneten Ebenen zugegeben wird, wobei eine Ebene in Hauptströmungsrichtung des Reaktionsgemischs stromauf und eine Ebene stromab zur Öffnung zur Zugabe des Amins angeordnet ist, so liegt das Verhältnis des Abstandes der Ebene, die stromab zur Öffnung, durch die das Amin zugegeben wird, angeordnet ist, zur Öffnung, durch die das Amin zugegeben wird, bezogen auf den Durchmesser der Mischkammer im Bereich von 0 bis 1, mehr bevorzugt im Bereich von 0,01 bis 0,5 und insbesondere im Bereich von 0,05 bis 0,2. Wenn das Phosgen durch Zufuhröffnungen in mehr als einer senkrecht zur Achse der Mischkammer angeordneten Ebene zugegeben wird, die in Hauptströmungsrichtung des Reaktionsgemischs stromab der Öffnung, durch die das Amin zugegeben wird, angeordnet sind, so ist der Abstand der Zufuhröffnungen der Ebene, die der Öffnung, über die das Amin zugegeben wird, am nächsten liegt, entsprechend dem Abstand der Ebene der Zufuhröffnungen, wenn nur eine Ebene stromab zur Öffnung, über die das Amin zugegeben wird, angeordnet ist.

Wenn das Phosgen durch Zufuhröffnungen in zwei senkrecht zur Achse der Mischkammer angeordneten Ebenen zugegeben wird, wobei eine Ebene in Hauptströmungsrichtung des Reaktionsgemischs stromauf und eine Ebene stromab zur Öffnung zur Zugabe des Amins angeordnet ist, so liegt das Verhältnis des Abstandes der Ebene, die stromauf zur Öffnung, durch die das Amin zugegeben wird, angeordnet ist, zur Öffnung, durch die das Amin zugegeben wird, bezogen auf den Durchmesser der Mischkammer im Bereich von 0 bis 1, mehr bevorzugt im Bereich von 0,01 bis 0,5 und insbesondere im Bereich von 0,05 bis 0,2. Wenn das Phosgen durch Zuführöffnungen in mehr als einer senkrecht zur Achse der Mischkammer angeordneten Ebene zugegeben wird, die in Hauptströmungsrichtung des Reaktionsgemischs stromauf der Öffnung, durch die das Amin zugegeben wird, angeordnet sind, so ist der Abstand der Zuführöffnungen der Ebene, die der Öffnung, über die das Amin zugegeben wird, am nächsten liegt, entsprechend dem Abstand der Ebene der Zuführöffnungen, wenn nur eine Ebene stromauf zur Öffnung, über die das Amin zugegeben wird, angeordnet ist.

Bevorzugt wird das Phosgen durch Zuführöffnungen in maximal fünf senkrecht zur Achse der Mischkammer angeordneten Ebenen zugegeben. Mehr bevorzugt ist es, wenn das Phosgen durch Zuführöffnungen in maximal drei senkrecht zur Achse der Mischkammer angeordnete Ebenen zugegeben wird und besonders bevorzugt, wenn das Phosgen durch Zuführöffnungen in zwei senkrecht zur Achse der Mischkammer angeordneten Ebenen zugegeben wird.

Die Anzahl der Zuführöffnungen in den einzelnen Ebenen ist vorzugsweise maximal fünf, mehr bevorzugt maximal vier und insbesondere zwei. Durch die Anzahl der Zuführöffnungen in den einzelnen Ebenen ergibt sich eine gute Verteilung des Phosgens in der Mischkammer. Hierbei ist es weiterhin bevorzugt, wenn die Öffnungen der einzelnen Ebenen gegeneinander verdreht sind, so dass die Zuführöffnungen der einzelnen Ebenen in Strömungsrichtung nicht fluchtend sind. Bei Zuführöffnungen in mehr als zwei Ebenen sind die Zuführöffnungen der einzelnen Ebenen vorzugsweise gleichmäßig zueinander gedreht. Der Winkel, um den die einzelnen Ebenen zueinander gedreht sind, errechnet sich dabei vorzugsweise zu

$$\alpha = \frac{180}{z_\delta},$$

wobei $\alpha$ der Winkel ist, um den die Ebenen zueinander gedreht sind und $z_\delta$ die Anzahl der Öffnungen je Ebene.

Der Durchmesser der Zuführöffnungen, durch die das Phosgen zugegeben wird, ist vorzugsweise kleiner als der Abstand der Ebenen, in denen die Zuführöffnungen angeordnet sind. Bevorzugt liegt der Durchmesser der Zuführöffnungen bezogen auf den Durchmesser der Mischkammer im Bereich von 0,01 bis 0,5, mehr bevorzugt im Bereich von 0,02 bis 0,3 und insbesondere im Bereich von 0,03 bis 0,25.

Die Zuführöffnungen können in jedem beliebigen Winkel in die Mischkammer münden. Vorzugsweise schneiden sich die Achsen der Zuführöffnungen mit der Achse der Mischkammer, besonders bevorzugt münden die Zuführöffnungen mit einem Winkel von 90° in die Mischkammer.

Die Zuführöffnungen, durch die das Phosgen zugegeben wird, sind vorzugsweise Düsenöffnungen. Das heißt, dass das Phosgen der Mischkammer über Leitungen zugeführt wird und am Ende der Leitungen eine Querschnittsverengung in Form einer Düse ausgebildet ist. Aus der Düse tritt das Phosgen dann in die Mischkammer ein. Die Zuführöffnung des Phosgens ist dabei vorzugsweise bündig mit der Wandung der Mischkammer. Die Düsen können sowohl kreisrunde als auch von der kreisrunden Form abweichende Öffnungen aufweisen.

Die Mischkammer, in der das Amin mit dem Phosgen vermischt wird, ist vorzugsweise rotationssymmetrisch. Wenn die Mischkammer keinen kreisförmigen Querschnitt hat, so ist als Durchmesser der Mischkammer immer der hydraulische Durchmesser gemeint.

An ihrem stromabwärtigen Ende weist die Mischkammer vorzugsweise eine Durchmesserverengung auf, durch die eine Rückvermischung des Reaktionsgemischs erfolgt. Die Rückvermischung ergibt sich durch die Umlenkung der Strömung aufgrund der Durchmesserverengung.

Die Durchmesserverengung am stromabwärtigen Ende der Mischkammer ist vorzugsweise mit einem Winkel im Bereich von 10 bis 80° zur Achse der Mischkammer ausgeführt. Mehr bevorzugt ist die Durchmesserverengung am stromabwärtigen Ende mit einem Winkel von 15 bis 60° und besonders bevorzugt mit einem Winkel von 18 bis 40° zur Achse der Mischkammer ausgeführt. Die Durchmesserverengung am stromabwärtigen Ende der Mischkammer ist vorzugsweise eine konische Verengung. Das Verhältnis des Durchmessers der Durchmesserverengung, auf die der Querschnitt reduziert wird, liegt bezogen auf den Durchmesser der Mischkammer im Bereich von 0,2 bis 0,7, mehr bevorzugt im Bereich von 0,25 bis 0,65 und insbesondere im Bereich von 0,3 bis 0,6. Neben einer Rückvermischung erfolgt durch die Durchmesserverengung somit auch eine Beschleunigung des Reaktionsgemischs.

Zur Strömungsvergleichmäßigung schließt sich an die Durchmesserverengung vorzugsweise eine Zone mit einem konstanten Durchmesser an, in der nur eine geringe Rückvermischung erfolgt.

Die Verweilzeit des Reaktionsgemischs in der Zone mit konstantem Durchmesser beträgt vorzugsweise maximal 50 ms, insbesondere maximal 30 ms. Die Länge der Zone mit konstantem Durchmesser bezogen auf den Durchmesser dieser Zone (UD-Verhältnis) liegt vorzugsweise im Bereich von 1 bis 10, mehr bevorzugt im Bereich von 1,5 bis 9 und insbesondere im Bereich von 2 bis 8.

An die Zone mit konstantem Durchmesser schließt sich eine Zone mit einer Querschnittserweiterung an, wobei die Querschnittserweiterung einen Öffnungswinkel bezogen auf die Achse der Zone aufweist, bei dem keine Ablösung der Strömung erfolgt. Das bedeutet, dass die Querschnittserweiterung in Form eines Diffusors ausgebildet ist. Die Querschnittserweiterung erweitert den Durchmesser, bis der Durchmesser des vorzugsweise als Rohrreaktor ausgebildeten Reaktors erreicht ist. Hierbei ist es möglich, dass der Durchmesser stufenweise erweitert wird, wobei sich zwischen die einzelnen Stufen, in denen der Durchmesser erweitert wird, jeweils ein Bereich mit konstantem Durchmesser angeordnet ist.

Um das Ablösen der Strömung zu verhindern, ist der Öffnungswinkel der Querschnittserweiterung zur Achse der Zone vorzugsweise kleiner als 15°, mehr bevorzugt kleiner als 10° und besonders bevorzugt kleiner als 8°.

Ein Ausführungsbeispiel der Erfindung ist anhand der Zeichnung dargestellt und wird in der nachfolgenden Beschreibung näher erläutert.

Die einzige Figur zeigt eine Vorrichtung zum Mischen von Amin und Phosgen in der Flüssigphase.

Bei Verfahren zur Herstellung von Isocyanaten durch Umsetzung der korrespondierenden Amine mit Phosgen in der Flüssigphase wird das Amin mit dem Phosgen vermischt, bevor die vermischten Edukte einem Reaktor, in dem die Umsetzung erfolgt, zugeführt werden.

Eine Vorrichtung zum Mischen von Amin und Phosgen umfasst eine Mischkammer 1, in die Phosgen und Amin zugeführt werden. Durch eine Öffnung 3, die koaxial zur Mischkammer 1 angeordnet ist, wird vorzugsweise das Amin zugegeben. Alternativ ist es jedoch auch möglich, dass durch die koaxial zur Mischkammer angeordnete Öffnung 3 das Phosgen zugeführt wird. Bevorzugt ist jedoch die Zugabe des Amins über die koaxial zur Mischkammer angeordnete Öffnung 3. Die koaxial zur Mischkammer 1 angeordnete Öffnung 3 ist z.B., wie hier dargestellt, in Form einer Düse ausgebildet, die in die Mischkammer 1 ragt.

Weiterhin umfasst die Vorrichtung zur Mischung von Phosgen und Amin Zufuhröffnungen 5, durch die das Phosgen bzw. bei Zugabe des Phosgens über die koaxial zur Achse der Mischkammer angeordnete Öffnung das Amin zugegeben wird. Die Zufuhröffnungen 5 sind ebenfalls vorzugsweise als Düsen ausgebildet. Die Zufuhröffnungen 5 sind in mindestens zwei Ebenen 7, 9, die senkrecht zur Achse der Mischkammer angeordnet sind, angeordnet. Die Ebenen 7, 9 sind hier durch gestrichelte Linien dargestellt. In der hier dargestellten Ausführungsform sind die Zufuhröffnungen 5 in zwei Ebenen 7, 9 angeordnet. Eine erste Ebene 7 ist dabei stromab zur koaxial angeordneten Öffnung 3 angeordnet und eine zweite Ebene 9 stromauf.

Neben der hier dargestellten Ausführungsform mit zwei Ebenen 7, 9, in denen die Zufuhröffnungen 5 angeordnet sind, ist es alternativ auch möglich, dass die Zufuhröffnungen in mehr als zwei Ebenen angeordnet sind. In dem Fall, dass die Zufuhröffnungen 5 in mehr als zwei Ebenen 7, 9 angeordnet sind, ist jeweils mindestens eine Ebene stromauf und mindestens eine Ebene stromab der koaxial angeordneten Öffnung 3 angeordnet.

Bevorzugt sind in jeder Ebene 7, 9 zwei Zufuhröffnungen 5 angeordnet, wobei sich die Zufuhröffnungen 5 jeweils diametral gegenüberliegen. Durch die Anordnung, bei der die Zufuhröffnungen 5 diametral gegenüberliegen, treffen sich die Hauptrichtungen der Zufuhröffnungen 5 in der Achse 11 der Mischkammer 1.

Das Verhältnis des Abstandes $L_1$ der ersten Ebene 7 zur koaxial zur Mischkammer angeordneten Öffnung 3 bezogen auf den Durchmesser $D_M$ der Mischkammer 1 liegt vorzugsweise im Bereich von 0 bis 1, mehr bevorzugt im Bereich von 0,01 bis 0,5 und insbesondere im Bereich von 0,05 bis 0,2. Wenn Zufuhröffnungen 5 in mehr als einer Ebene stromab zur koaxial zur Mischkammer angeordneten Öffnung 3 angeordnet sind, so ist dies der Abstand der Ebene, die der koaxial zur Mischkammer angeordneten Öffnung 3 am nächsten liegt.

Das Verhältnis des Abstandes $L_2$ der zweiten Ebene 9, die stromauf zur koaxial zur Mischkammer 1 angeordneten Öffnung 3 angeordnet ist, bezogen auf den Durchmesser $D_M$ der Mischkammer 1 liegt ebenfalls vorzugsweise im Bereich von 0 bis 1, mehr bevorzugt im Bereich von 0,01 bis 0,5 und insbesondere im Bereich von 0,05 bis 0,2. Wenn Zufuhröffnungen 5 in mehr als zwei Ebenen stromauf zur koaxial zur Mischkammer 1 angeordneten Öffnung 3 angeordnet sind, so entspricht dies dem Abstand der Ebene, die der Öffnung 3 am nächsten liegt.

An ihrem stromabwärtigen Ende weist die Mischkammer 1 eine Durchmesserverengung 13 auf. Die Durchmesserverengung 13 ist vorzugsweise konisch ausgebildet und mit einem Winkel $\alpha$ im Bereich von 10 bis 80°, bevorzugt mit einem Winkel im Bereich von 15 bis 60° und besonders bevorzugt mit einem Winkel von 18 bis 40° zur Achse 11 der Mischkammer 1 ausgeführt.

An die Durchmesserverengung 13 schließt sich eine Zone konstanten Durchmessers 15 an. Die Zone 15 konstanten Durchmessers weist einen Durchmesser $D_A$ auf, wobei das Verhältnis des Durchmessers $D_A$ der Zone 15 konstanten Durchmessers zum Durchmesser $D_M$ der Mischkammer 1 wie vorstehend bereits beschrieben im Bereich von 0,2 bis 0,7, mehr bevorzugt im Bereich von 0,25 bis 0,65 und insbesondere im Bereich von 0,3 bis 0,6 liegt. Mit der Durchmesserverengung 13 nimmt der Durchmesser vom Durchmesser $D_M$ der Mischkammer 1 auf den Durchmesser $D_A$ der Zone 15 konstanten Durchmessers ab.

An die Zone 15 konstanten Durchmessers schließt sich eine Querschnittserweiterung 17 an. Die Querschnittserweiterung 17 ist dabei vorzugsweise in Form eines Diffusors ausgebildet. Die Querschnittserweiterung 17 weist einen Öffnungswinkel $\beta$ auf, der so ausgewählt ist, dass in der Querschnittserweiterung 17 keine Ablösung der Strömung auftritt. Alternativ zur hier dargestellten Ausführungsform mit einer sich konisch erweiternden Querschnittserweiterung 17 ist es z.B. auch möglich, dass der Durchmesser in der Querschnittserweiterung 17 stufenweise erweitert wird. In diesem Fall ist zwischen den einzelnen Stufen, in denen der Durchmesser erweitert wird, jeweils ein Bereich mit konstantem Durchmesser angeordnet. Alternativ ist es auch möglich, dass zwischen den einzelnen Stufen jeweils ein Bereich ausgebildet ist, in dem sich der Durchmesser konisch erweitert.

Besonders bevorzugt ist die Querschnittserweiterung 17 jedoch konisch ausgebildet und der Öffnungswinkel β der Querschnittserweiterung 17 ist vorzugsweise < 15°, mehr bevorzugt < 10° und besonders bevorzugt < 8°.

Die Länge der Querschnittserweiterung 17 ist so gewählt, dass sich der Durchmesser auf den Durchmesser des sich an die Vorrichtung zur Vermischung des Amins und Phosgens anschließenden Reaktors erweitert, der hier nicht dargestellt ist.

Um eine kurze Verweilzeit und hohe Mischgeschwindigkeiten in der Mischkammer 1 zu erzielen, ist das Verhältnis der Länge $L_M$ der Mischkammer 1 bezogen auf den Durchmesser $D_M$ vorzugsweise im Bereich zwischen 1 und 2 und insbesondere im Bereich von 1 bis 1,5. Das Verhältnis der Länge $L_A$ der Zone 15 konstanten Durchmessers bezogen auf den Durchmesser $D_A$ der Zone konstanten Durchmessers liegt vorzugsweise im Bereich von 1 bis 10, mehr bevorzugt im Bereich von 1,5 bis 9 und insbesondere im Bereich von 2 bis 8.

Beispiel

Zur Herstellung von MDI/PMDI wird eine Vorrichtung eingesetzt, die eine Mischkammer mit einem Durchmesser von 40 mm und einer Länge von 66 mm umfasst. In die Mischkammer mündet eine Zuführungseinrichtung für das Amin, die 26 mm in die Mischkammer hineinragt, mit einem Durchmesser von 20 mm und einem Düsendurchmesser von 5,5 mm. Zur Zufuhr des Phosgen sind zwei Zufuhröffnungen diametral 6 mm oberhalb vom Austrittsquerschnitt der zentralen Düse und zwei Zufuhröffnungen diametral 6 mm unterhalb vom Austrittsquerschnitt der zentralen Düse angeordnet. Der Durchmesser der Zufuhröffnungen oberhalb des Austrittsquerschnitts der zentralen Düse beträgt 5,1 mm bei einem Durchmesser der Zufuhreinrichtung von 15 mm und der Düsendurchmesser der Zufuhröffnungen unterhalb, d.h. stromab der Öffnung der zentralen Düse, beträgt 6,9 mm bei einem Durchmesser der Zufuhreinrichtung von 20 mm.

Die Mischkammer weist eine konische Verengung mit einem Winkel von 25° auf, wobei der Durchmesser vom Mischkammerdurchmesser von 40 mm auf den Austrittsdurchmesser von 25 mm abnimmt. Die Gesamtlänge der Mischkammer umfassend den zylindrischen Teil und den konischen Teil beträgt 66 mm. An die Mischkammer schließt sich eine Zone konstanten Durchmessers mit einer Länge von 180 mm an. An die Zone konstanten Durchmessers schließt sich eine Erweiterung mit einem Öffnungswinkel von 6° an. Mit der Erweiterung nimmt der Durchmesser auf den Durchmesser des nachfolgenden Rohrreaktors zu.

Über die zentrale Düse werden 3,75 m$^3$/h eines aminhaltigen Stoffstroms und über die Zufuhröffnungen 11,2 m$^3$/h eines phosgenhaltigen Stroms zugeführt. Der aminhaltige Strom enthält 34 bis 36 Gew.-% MDA/PMDA mit einem Anteil von 50,4 bis 51,1 Gew.-% MDA und 54 bis 56 Gew.-% Monochlorbenzen und der phosgenhaltige Strom enthält 66 bis 70 Gew.-% Phosgen und 30 bis 34 Gew.-% Monochlorbenzen.

Die Verweilzeit in der Mischzone beträgt 17 ms. Die Verweilzeit in der Zone mit konstantem Durchmesser beträgt ungefähr 21 ms.

Bezugszeichenliste

| 1 | Mischkammer |
|---|---|
| 3 | Öffnung koaxial zur Mischkammer |
| 5 | Zufuhröffnung |
| 7 | erste Ebene |
| 9 | zweite Ebene |
| 11 | Achse |
| 13 | Durchmesserverengung |
| 15 | Zone konstanten Durchmessers |
| 17 | Querschnittserweiterung |
| $D_A$ | Durchmesser der Zone 15 konstanten Durchmessers |
| $D_M$ | Durchmesser der Mischkammer 1 |

(fortgesetzt)

| | |
|---|---|
| $L_A$ | Länge der Zone 15 konstanten Durchmessers |
| $L_M$ | Länge der Mischkammer 1 |
| $L_1$ | Abstand der ersten Ebene 7 zur Öffnung 3 |
| $L_2$ | Abstand der zweiten Ebene 9 zur Öffnung 3 |
| $\alpha$ | Winkel in dem die Durchmesserverengung 13 ausgeführt ist |
| $\beta$ | Öffnungswinkel der Querschnittserweiterung 17 |
| | |
| | |
| | |
| | |
| | |
| | |

**Patentansprüche**

1. Verfahren zur Herstellung von Isocyanaten durch Umsetzung der korrespondierenden Amine mit Phosgen in der Flüssigphase, gegebenenfalls in Gegenwart mindestens eines Inertmediums, bei dem zunächst das Amin und das Phosgen in einer Mischkammer (1) zu einem Reaktionsgemisch gemischt werden und das Reaktionsgemisch einem Reaktor zugeführt wird, wobei das Amin durch eine koaxial zur Mischkammer (1) angeordnete Öffnung (3) zugegeben wird und das Phosgen durch Zufuhröffnungen (5) in mindestens zwei senkrecht zur Achse der Mischkammer angeordneten Ebenen (7, 9) zugegeben wird oder das Phosgen durch die koaxial zur Mischkammer angeordnete Öffnung (3) und das Amin durch die Zufuhröffnungen (5) in mindestens zwei senkrecht zur Achse (11) der Mischkammer (1) angeordneten Ebenen (7, 9) zugegeben werden, wobei mindestens eine Ebene (9) in Hauptströmungsrichtung des Reaktionsgemischs stromauf und mindestens eine Ebene (7) stromab zur Öffnung (3) zur Zugabe des Amins angeordnet ist, **dadurch gekennzeichnet, dass** die mittlere Verweilzeit des Reaktionsgemischs in der Mischkammer (1) maximal 18,5 ms beträgt.

2. Verfahren gemäß Anspruch 1, **dadurch gekennzeichnet, dass** das Phosgen über jeweils mindestens zwei Zufuhröffnungen (5) in den mindestens zwei senkrecht zur Achse der Mischkammer angeordneten Ebenen (7, 9) zugegeben wird.

3. Verfahren gemäß Anspruch 2, **dadurch gekennzeichnet, dass** die Zufuhröffnungen (5), durch die das Phosgen zugegeben wird, so angeordnet sind, dass sich die Hauptrichtungen der Zufuhröffnungen (5) in der Achse (11) der Mischkammer (1) treffen.

4. Verfahren gemäß einem der Ansprüche 1 bis 3, **dadurch gekennzeichnet, dass** die Mischkammer (1) an ihrem stromabwärtigen Ende eine Durchmesserverengung (13) aufweist, durch die eine Rückvermischung des Reaktionsgemischs erfolgt.

5. Verfahren gemäß Anspruch 4, **dadurch gekennzeichnet, dass** die Durchmesserverengung (13) am stromabwärtigen Ende der Mischkammer (1) mit einem Winkel ($\alpha$) im Bereich von 10 bis 80° zur Achse (11) der Mischkammer (1) ausgeführt ist.

6. Verfahren gemäß einem der Ansprüche 1 bis 5, **dadurch gekennzeichnet, dass** sich an die Mischkammer (1) eine Zone (15) mit einem konstanten Durchmesser anschließt, in der nur eine geringe Rückvermischung erfolgt.

7. Verfahren gemäß Anspruch 6, **dadurch gekennzeichnet, dass** die Verweilzeit des Reaktionsgemischs in der Zone (15) mit konstantem Durchmesser maximal 50 ms beträgt.

8. Verfahren gemäß Anspruch 6 oder 7, **dadurch gekennzeichnet, dass** sich an die Zone (15) mit konstantem Durchmesser eine Zone mit einer Querschnittserweiterung (17) anschließt, wobei die Querschnittserweiterung (17) einen Öffnungswinkel (β) aufweist, bei dem keine Ablösung der Strömung erfolgt.

9. Verfahren gemäß Anspruch 8, **dadurch gekennzeichnet, dass** der Öffnungswinkel (β) der Querschnittserweiterung (17) zur Achse (11) der Zone mit der Querschnittserweiterung (17) kleiner ist als 15°.

10. Vorrichtung zur Herstellung von Isocyanaten durch Umsetzung der korrespondierenden Amine mit Phosgen in der Flüssigphase, gegebenenfalls in Gegenwart mindestens eines Inertmediums, umfassend eine Mischkammer (1) zur Mischung des Amins und des Phosgens zu einem Reaktionsgemisch, wobei in die Mischkammer (1) eine koaxial zur Mischkammer (1) angeordnete Öffnung (3) mündet und weiterhin Zufuhröffnungen (5) in mindestens zwei senkrecht zur Achse (11) der Mischkammer (1) angeordneten Ebenen (7, 9) in die Mischkammer (1) münden, wobei mindestens eine Ebene (9) in Hauptströmungsrichtung des Reaktionsgemischs stromauf und mindestens eine Ebene (7) stromab zur koaxial zur Mischkammer (1) angeordneten Öffnung (3) angeordnet ist, wobei die Mischkammer (1) an ihrem stromabwärtigen Ende eine Durchmesserverengung (13) aufweist, die mit einem Winkel (α) im Bereich von 10 bis 80° zur Achse (11) der Mischkammer (1) ausgeführt ist.

11. Vorrichtung gemäß Anspruch 10, **dadurch gekennzeichnet, dass** sich an die Durchmesserverengung (13) eine Zone (15) konstanten Durchmessers anschließt.

12. Vorrichtung gemäß Anspruch 10 oder 11, **dadurch gekennzeichnet, dass** sich an die Zone (15) mit konstantem Durchmesser eine Zone mit einer Querschnittserweiterung (17) anschließt, wobei die Querschnittserweiterung (17) einen Öffnungswinkel (β) aufweist, bei dem keine Ablösung der Strömung erfolgt.

13. Vorrichtung gemäß Anspruch 12, **dadurch gekennzeichnet, dass** der Öffnungswinkel (β) der Querschnittserweiterung (17) zur Achse (11) der Zone mit der Querschnittserweiterung kleiner ist als 15°.

**Claims**

1. A process for preparing isocyanates by reacting the corresponding amines with phosgene in the liquid phase, optionally in the presence of at least one inert medium, in which the amine and the phosgene are first mixed in a mixing chamber (1) to give a reaction mixture and the reaction mixture is fed to a reactor, the amine being added through an orifice (3) arranged coaxially to the mixing chamber (1) and the phosgene being added through feed orifices (5) in at least two planes (7, 9) arranged at right angles to the axis of the mixing chamber, or the phosgene being added through the orifice (3) arranged coaxially to the mixing chamber and the amine through the feed orifices (5) in at least two planes (7, 9) arranged at right angles to the axis (11) of the mixing chamber (1), at least one plane (9) being arranged upstream and at least one plane (7) downstream of the orifice (3) for addition of the amine in main flow direction of the reaction mixture, wherein the mean residence time of the reaction mixture in the mixing chamber (1) is not more than 18.5 ms.

2. The process according to claim 1, wherein the phosgene is added via in each case at least two feed orifices (5) in the at least two planes (7, 9) arranged at right angles to the axis of the mixing chamber.

3. The process according to claim 2, wherein the feed orifices (5) through which the phosgene is added are arranged such that the main directions of the feed orifices (5) meet in the axis (11) of the mixing chamber (1).

4. The process according to any one of claims 1 to 3, wherein the mixing chamber (1) has, at its downstream end, a diameter constriction (13) by virtue of which the reaction mixture is backmixed.

5. The process according to claim 4, wherein the diameter constriction (13) at the downstream end of the mixing chamber (1) is configured with an angle (α) in the range from 10 to 80° relative to the axis (11) of the mixing chamber (1).

6. The process according to according to any one of claims 1 to 5, wherein the mixing chamber (1) is followed downstream by a zone (15) with a constant diameter in which there is only minor backmixing.

7. The process according to claim 6, wherein the residence time of the reaction mixture in the zone (15) with constant diameter is not more than 50 ms.

8. The process according to claim 6 or 7, wherein the zone (15) with constant diameter is followed downstream by a zone with a cross-sectional enlargement (17), the cross-sectional enlargement (17) having an opening angle (β) at which there is no discontinuity of flow.

9. The process according to claim 8, wherein the opening angle (β) of the cross-sectional enlargement (17) relative to the axis (11) of the zone with the cross-sectional enlargement (17) is less than 15°.

10. An apparatus for preparing isocyanates by reacting the corresponding amines with phosgene in the liquid phase, optionally in the presence of at least one inert medium, comprising a mixing chamber (1) for mixing the amine and the phosgene to give a reaction mixture, an orifice (3) arranged coaxially to the mixing chamber (1) opening into the mixing chamber (1) and feed orifices (5) also opening into the mixing chamber (1) in at least two planes (7, 9) arranged at right angles to the axis (11) of the mixing chamber (1), at least one plane (9) being arranged upstream and at least one plane (7) downstream of the orifice (3) arranged coaxially to the mixing chamber (1) in main flow direction of the reaction mixture, the mixing chamber (1) having, at its downstream end, a diameter constriction (13) which is configured with an angle (α) in the range from 10 to 80° relative to the axis (11) of the mixing chamber (1).

11. The apparatus according to claim 10, wherein the diameter constriction (13) is followed downstream by a zone (15) of constant diameter.

12. The apparatus according to claim 10 or 11, wherein the zone (15) with constant diameter is followed downstream by a zone with a cross-sectional enlargement (17), the cross-sectional enlargement (17) having an opening angle (β) at which there is no discontinuity of flow.

13. The apparatus according to claim 12, wherein the opening angle (β) of the cross-sectional enlargement (17) relative to the axis (11) of the zone with the cross-sectional enlargement is less than 15°.


**Revendications**

1. Procédé de fabrication d'isocyanates par mise en réaction des amines correspondantes avec du phosgène en phase liquide, éventuellement en présence d'au moins un milieu inerte, selon lequel l'amine et le phosgène sont tout d'abord mélangés dans une chambre de mélange (1) pour former un mélange réactionnel et le mélange réactionnel est introduit dans un réacteur, l'amine étant introduite par une ouverture (3) agencée coaxialement à la chambre de mélange (1) et le phosgène étant introduit par des ouvertures d'alimentation (5) dans au moins deux plans (7, 9) agencés perpendiculairement à l'axe de la chambre de mélange ou le phosgène étant introduit par l'ouverture (3) agencée coaxialement à la chambre de mélange et l'amine étant introduite par les ouvertures d'alimentation (5) dans au moins deux plans (7, 9) agencés perpendiculairement à l'axe (11) de la chambre de mélange (1), au moins un plan (9) étant agencé en amont dans la direction principale d'écoulement du mélange réactionnel et au moins un plan (7) en aval de l'ouverture (3) pour l'introduction de l'amine, **caractérisé en ce que** le temps de séjour moyen du mélange réactionnel dans la chambre de mélange (1) est d'au plus 18,5 ms.

2. Procédé selon la revendication 1, **caractérisé en ce que** le phosgène est introduit par à chaque fois au moins deux ouvertures d'alimentation (5) dans les deux plans (7, 9) ou plus agencés perpendiculairement à l'axe de la chambre de mélange.

3. Procédé selon la revendication 2, **caractérisé en ce que** les ouvertures d'alimentation (5) par lesquelles le phosgène est introduit sont agencées de manière à ce que les directions principales des ouvertures d'alimentation (5) se croisent dans l'axe (11) de la chambre de mélange (1).

4. Procédé selon l'une quelconque des revendications 1 à 3, **caractérisé en ce que** la chambre de mélange (1) présente à son extrémité aval un rétrécissement de diamètre (13), qui provoque un rétromélange du mélange réactionnel.

5. Procédé selon la revendication 4, **caractérisé en ce que** le rétrécissement de diamètre (13) à l'extrémité aval de la chambre de mélange (1) est configuré avec un angle (α) dans la plage allant de 10 à 80° par rapport à l'axe (11) de la chambre de mélange (1).

6. Procédé selon l'une quelconque des revendications 1 à 5, **caractérisé en ce qu'**une zone (15) de diamètre constant, où seul un faible rétromélange a lieu, suit la chambre de mélange (1).

7.  Procédé selon la revendication 6, **caractérisé en ce que** le temps de séjour du mélange réactionnel dans la zone (15) de diamètre constant est d'au plus 50 ms.

8.  Procédé selon la revendication 6 ou 7, **caractérisé en ce qu'**une zone présentant un élargissement de section (17) suit la zone (15) de diamètre constant, l'élargissement de section (17) présentant un angle d'ouverture (β) auquel aucun décollement de l'écoulement n'a lieu.

9.  Procédé selon la revendication 8, **caractérisé en ce que** l'angle d'ouverture (β) de l'élargissement de section (17) par rapport à l'axe (11) de la zone présentant l'élargissement de section (17) est inférieur à 15°.

10.  Dispositif pour la fabrication d'isocyanates par mise en réaction des amines correspondantes avec du phosgène en phase liquide, éventuellement en présence d'au moins un milieu inerte, comprenant une chambre de mélange (1) pour le mélange de l'amine et du phosgène pour former un mélange réactionnel, une ouverture (3) agencée coaxialement à la chambre de mélange (1) débouchant dans la chambre de mélange (1) et des ouvertures d'alimentation (5) dans au moins deux plans (7, 9) agencés perpendiculairement à l'axe (11) de la chambre de mélange (1) débouchant également dans la chambre de mélange (1), au moins un plan (9) étant agencé en amont dans la direction principale d'écoulement du mélange réactionnel et au moins un plan (7) en aval de l'ouverture (3) agencée coaxialement à la chambre de mélange (1), la chambre de mélange (1) présentant à son extrémité aval un rétrécissement de diamètre (13), qui est configuré avec un angle (α) dans la plage allant de 10 à 80° par rapport à l'axe (11) de la chambre de mélange (1).

11.  Dispositif selon la revendication 10, **caractérisé en ce qu'**une zone (15) de diamètre constant suit le rétrécissement de diamètre (13).

12.  Dispositif selon la revendication 10 ou 11, **caractérisé en ce qu'**une zone présentant un élargissement de section (17) suit la zone (15) de diamètre constant, l'élargissement de section (17) présentant un angle d'ouverture (β) auquel aucun décollement de l'écoulement n'a lieu.

13.  Dispositif selon la revendication 12, caractérisé en ce l'angle d'ouverture (β) de l'élargissement de section (17) par rapport à l'axe (11) de la zone présentant l'élargissement de section est inférieur à 15°.

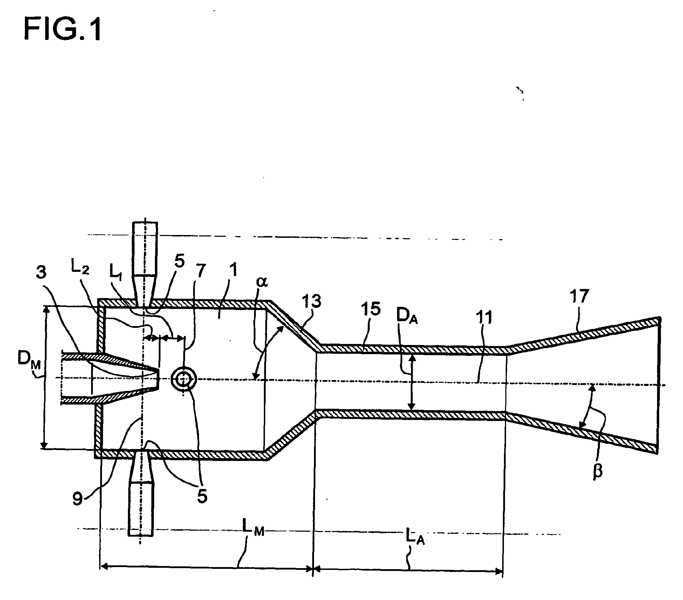

FIG.1

EP 2 323 973 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DD 300168 A